# EUROPEAN PATENT APPLICATION

(11) **EP 4 094 791 A1**
(43) Date of publication of application: **30.11.2022**
(21) Application number: 21305693.0
(22) Date of filing: 26.05.2021
(51) Int. Cl.: A61M 5/31, A61M 5/315, A61M 5/32, H05K 1/00, H05K 3/00, A61J 1/05, G06K 19/07

(54) **MEDICAL DEVICE WITH RFID TAG DIRECTLY PRINTED ON ONE PART OF THIS MEDICAL DEVICE**

(71) Applicant: Becton Dickinson France, 38800 Le Pont-de-Claix (FR)
(72) Inventor: GARREC, Ronan, 38640 Claix (FR); LEIBBRAND, Alfred, 38801 Le Pont de Claix (FR); RIVIER, Cédric, 38340 Voreppe (FR); LUXEMBOURG, David, 38000 Grenoble (FR)
(74) Representative: Germain Maureau

(57) **Abstract**

The present invention relates to a medical component comprising a Radio Frequency Identification RFID tag (20, 30, 40) located on the component, said RFID tag (20, 30, 40) comprising at least one RFID antenna (22, 32, 42), the antenna being made of a substantially transparent and conductive ink.

The present invention also relates to a medical injection device comprising a medical component according to the present invention.

The present invention finally relates to a method for manufacturing a medical component according to the present invention.

## Description

### Field of the invention

The present invention relates to a medical injection device and a method for manufacturing said medical injection device. The invention is particularly well suited for the healthcare industry.

### Background art

In this application, the distal end of a component or of a device is to be understood as meaning the end furthest from the user's hand and the proximal end is to be understood as meaning the end closest to the user's hand. Likewise, in this application, the "distal direction" is to be understood as meaning the direction of injection, with respect to a medical container of the invention, and the "proximal direction" is to be understood as meaning the opposite direction to said direction of injection, that is to say the direction towards the user's hand holding a container as for an injection operation.

Medical injection devices, for example pre-fillable or prefilled syringes, usually comprise a hollow body or barrel forming a container for a medical product. This body comprises a distal end, optionally provided with a needle, and a proximal end, usually provided with a flange.

The body has usually a substantially cylindrical shape, and comprises a proximal end sealed by a plunger stopper, a distal end with a distal hub wherein the medical product is expelled from the container, and a wall extending between the proximal end and the distal end. In practice, the plunger stopper aims at moving, upon a pressure exerted by a plunger rod, from a proximal end of the barrel towards the distal end of the barrel, or from a distal end of the barrel towards the proximal end of the barrel thereby expelling the drug contained into a patient body or withdrawing a liquid from a vial or from a patient body.

Medical injection devices also include empty injection devices that are filled with a vial-stored pharmaceutical composition just prior to the injection to the patient's body.

There is an increasing need for individual traceability of medical containers, such as medical injection devices, from the manufacturing process until the final labeling, the final use or the disposal of said medical containers. Indeed, medical injection devices are usually not processed individually but may be carried or packed with a plurality of similar medical injection devices, the traceability should ensure that the status of the medical containers may be checked at any time during the life cycle of the medical injection devices. The traceability should also ensure that the final user knows exactly what is the medical composition contained in the medical injection device, and prevent any mix-up of containers.

It is known, for example, from WO2017157784, a receptacle having a cylindrical lateral surface surrounded by a sequence of printed machine-readable unique identifier codes. These printed unique identifier codes allow tracking of each receptacle along a supply chain. However, the unique identifier codes cover a portion of the receptacle so that they may have an impact on a user visual inspection process.

In this context, an object of the present invention is to provide a device that alleviates the above-mentioned drawbacks by allowing an effective individual identification of a medical injection device with few impact on visual inspection, with few or no risks of being removed or damaged, and with a limited impact on the manufacturing process.

### Summary of the invention

A first aspect of the present invention is a medical component comprising a Radio Frequency Identification (RFID) tag located on the component, said RFID tag comprising at least one RFID antenna being made of a substantially transparent and conductive ink.

Without willing to be bound by any theory, it is believed that the medical injection device of the invention allows individual traceability of each medical injection device from the manufacturing process to the final use of the medical injection device. Besides, the thin thickness of the RFID tag only slightly affects the outer wall of the medical injection, i.e. the external dimension of the medical injection device. Thus, the medical injection device is compatible with medical devices such as auto-injector systems or safety devices. Moreover, no change is required regarding the packaging or storing of the medical injection device. Furthermore, the RFID tag comprising the at least one RFID antenna made of a substantially transparent and conductive ink allows a limited visual impact on the medical injection device. Typically the RFID antenna comprises two extremities. Therefore, the visual inspection of the medical injection device can be easily performed. Finally, the application of the RFID tag has only a limited impact on the cover manufacturing process.

In this application, a transparent ink allows at least 90% of light transmission in the visible, and more preferably allows at least 98% of light transmission in the visible as measured by a spectrophotometer lambda 900. In this application, an opaque material absorbs more than 99%, preferably more than 100% of the light.

In one embodiment, the medical component is chosen from: a barrel having a hub portion provided at its distal end, a plunger rod, a plunger stopper and a cover.

In one embodiment, the RFID tag does not comprise a chip. Such a chipless RFID tag does not require a chip to be read by a reader.

In one embodiment, a chip is connected to the at least one RFID antenna being made of a substantially transparent and conductive ink. Preferably in this embodiment the RFID antenna is bonded to both extremities of the RFID antenna.

In one embodiment, the RFID tag extends along a longitudinal axis of said medical component.

In one embodiment, the RFID tag extends along a transversal axis of said component.

Advantageously, the RFID tag has a length extending strictly less than 100% of a length of the component. Preferably, the RFID tag has a length extending over at least 15%, typically at least 50%, preferably at least 70% of a length of said component. It is believed that the length of the tag can have an impact on the data transmission level of the RFID tag to the RFID reader: the longer the tag, the better the data transmission.

Advantageously, the RFID tag has a width extending between 10% and 100%, 100% being preferably excluded, preferably between 40 and 100%, more preferably between 50% and 100%, advantageously between 50% and 90% and for example between 40 and 90% of the circumference of an external wall of the medical component. In this embodiment, it is believed that if the RFID tag overlaps the circumference of the outer wall of the component, there is a risk to decrease the data transmission level to the RFID reader.

In one embodiment, the RFID tag is located at a proximal end of said component.

In one embodiment, the RDIF tag is applied on a transparent substrate. Preferably, the substrate is made of plastic.

In one embodiment, the RFID tag is directly covered by a protective transparent coating. Advantageously, the protective coating is made of an epoxy resin such as an epoxy varnish. In this embodiment, it is believed that the protective transparent coating further decreases the risk of removal or external damage.

In one embodiment, the RFID chip is a printed circuit.

In one embodiment, the RFID chip has a length strictly less than 0.3mm, a width strictly less than 0.3mm and a thickness strictly less than 0.08mm. Preferably, the RFID chip has a length equal or below 0.25mm, a width equal or below 0.25mm and a thickness equal or below 0.055mm. In this embodiment, it is believed that even though the RFID chip is opaque, thanks to the small size of the RFID chip, the visual impact of the medical injection is limited.

In one embodiment, the RFID tag is a Low Frequency Radio Frequency Identification (LF-RFID) tag. Low frequencies are usually about 30 KHz to 300 KHz. In this embodiment, a RFID reader can for example read the LF-RFID tag at a distance up to about 10 cm.

In one embodiment, the RFID tag is a High Frequency Radio Frequency Identification (HF-RFID) tag. High frequencies are usually about 1-15 MHz. In this embodiment, a RFID reader can for example read the HF-RFID tag at a distance about one meter.

Preferably, the RFID tag is an Ultra High Frequency Radio Frequency Identification (UHF-RFID) tag. Ultra high frequencies are usually about 400-1000MHz. In this embodiment, a RFID reader can for example read the UHF-RFID tag at a distance about fifteen meters.

A second aspect of the present invention is a medical injection device comprising a medical component according to the present invention.

In one embodiment, the medical injection device is a syringe.

A third aspect of the present invention is a method for manufacturing a medical component according to the present invention or a medical injection device according to the present invention, comprising a step A) wherein a RFID tag comprising at least one RFID antenna made of a transparent and conductive ink is applied onto a medical component, preferably chosen among: a barrel having a hub portion, a plunger rod, a barrel stopper and a cover.

In one embodiment, the step A) comprises the following steps:
I. The provision of the medical component and
II. The application onto the medical component of the RFID tag comprising at least one RFID antenna made of a transparent and conductive ink.

In one embodiment, in step II), the application of the at least one RFID antenna made of a transparent and conductive ink onto the component is performed by printing, in particular by screen-printing or ink-jet printing; by roll coating; by blade coating; by rod coating, by spin coating or pad-printing or by slot-die coating.

Preferably, the application of the at least one RFID antenna made of a transparent and conductive ink on the component is performed by printing, in particular by screen-printing or ink-jet printing. In this embodiment, it is believed that the printing allows for an optimized application of the RFID antenna.

For example, the transparent and conductive ink comprises silver nanowires such as PolyBioWire^{®} or graphene such as Vor-ink^{®}, preferably PolyBioWire^{®}.

In one embodiment, the method further comprises a step II') performed after step II), wherein a RFID chip is bonded to said at least one RFID antenna made of a transparent and conductive ink.

In one embodiment, the bonding of the RFID chip on said at least one RFID antenna made of a transparent and conductive ink is performed by gluing, by brazing or by welding. Preferably, the bonding of the RFID chip on said at least one RFID antenna made of a transparent and conductive ink is performed by gluing. The glue can be a conductive and anisotropic epoxy glue such as Deleo monopox glue. In this embodiment, it is believed that there is no risk that the manufacturing process damage the medical injection device. In this embodiment, the chip preferably needs to be bonded firmly at the two extremities of the antenna.

In one embodiment, the method comprises a step III) wherein a protective transparent coating is suitably applied onto the RFID tag by serigraphy.

In one embodiment, the step A) comprises the following steps:
i) the provision of a transparent substrate;
ii) the application onto the transparent substrate of the RFID tag comprising at least one RFID antenna made of a transparent and conductive ink;
iii) the provision of the medical component and
iv) the application onto the medical component of the transparent substrate comprising the RFID tag comprising at least one RFID antenna made of a transparent and conductive ink.

In one embodiment, in step ii), the application of the at least one RFID antenna made of a transparent and conductive ink onto the transparent substrate is performed by printing, in particular by screen-printing or ink-jet printing; by roll coating; by blade coating; by rod coating, by spin coating or pad-printing or by slot-die coating.

In one embodiment, the method further comprises a step ii') performed before step iii), wherein a RFID chip is bonded to said at least one RFID antenna made of a transparent and conductive ink.

In one embodiment, the bonding of the RFID chip to said at least one RFID antenna made of a transparent and conductive ink is performed by gluing, by brazing or by welding.

In one embodiment, in step iv), the RFID tag comprising at least one RFID antenna made of a transparent and conductive ink is implemented onto the component by over molding or in mold labelling.

In one embodiment, the method further comprises a step v) wherein a protective transparent coating is applied onto the RFID tag by serigraphy.

### Brief description of the drawings

The invention and advantages arising therefrom will clearly emerge from the detailed description that is given below with reference to the appended drawings as follows:
Figure 1 is a transversal view of a medical injection device according to one embodiment of the invention;
Figure 2 is a perspective view of a barrel according to the embodiment illustrated in Figure 1;
Figure 3 is a cross-sectional view of a barrel according to the embodiment illustrated in Figures 1 and 2;
Figure 4 is a perspective view of a barrel according to another embodiment;
Figure 5 is a transversal view of a medical injection device furnished with a cover according to another embodiment of the invention and
Figure 6 is a perspective view of a cover according to the embodiment illustrated in Figure 4.

### Detailed description of the drawings

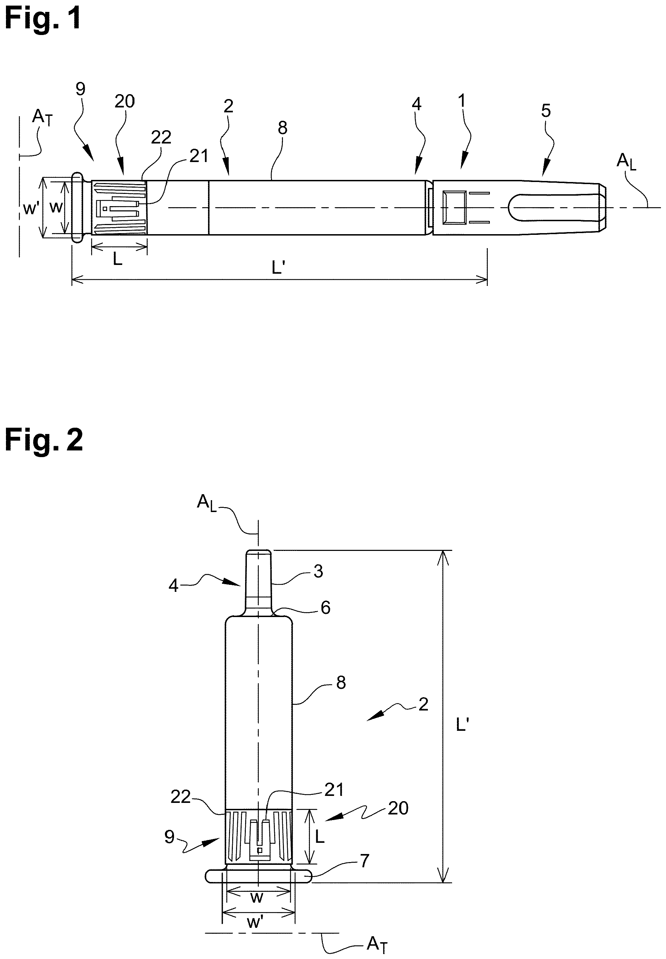

Figures 1 to 4 show a medical injection device 1 such as a syringe comprising a barrel 2 having a longitudinal axis A_{L} and a transversal axis A_{T}. Said barrel 2 comprises a side wall 8 and thus forms a reservoir, adapted to contain a medical composition to be injected.

The medical injection device 1 further comprises a hub portion 3 provided at its distal end 4 and extending along the axis A_{L} from the distal end of the barrel 2. The hub portion 3 is partially hollow so as to form a channel in fluidic communication with the barrel 2.

A needle (not shown) may be attached to the hub portion 3 of the medical injection device. For example, the needle may be glued to the hub portion 3. A cover 5 is intended to cover the hub portion 3 of the medical injection device 1, so as to protect the optional needle. The medical injection device 1 can also include, at its distal end, a distal shoulder 6 which narrows with respect to the barrel 2.

The medical injection device or syringe 1 shown in Figure 1 also may include a plunger rod (not shown) having a plunger (not shown) and a barrel stopper (not shown) provided at an end thereof. The plunger is caused to slidably move in the barrel 2 along an inner surface of the side wall 8 to cause the medical composition to be expelled from the barrel 2 through the needle. The medical composition comprised in the medical injection device 1 may be for example, a liquid medicament, a drug or a pharmaceutical composition such as a vaccine. The barrel 2 may also include a flange 7 located at the proximal end of the barrel.

In Figures 1 to 4, the medical component of the invention is the barrel 2. For example, the barrel 2 is entirely transparent. Preferably, the barrel 2 is made of glass or plastic such as cyclic olefin copolymer (COC) or Cyclo Olefin Polymer (COP).

As shown in Figures 1 and 2, the barrel 2 comprises a RFID tag 20 located on the barrel 2, said RFID tag 20 comprising at least one RFID antenna 22 made of a substantially transparent and conductive ink. The RFID tag 20 may comprise a RFID chip 21 connected to the at least one RFID antenna 22. Said RFID antenna 22 preferably comprise two extremities. When the RFID tag 20 comprises a RFID chip 21, said RFID 21 is bonded to both extremities of the RFID antenna 22.

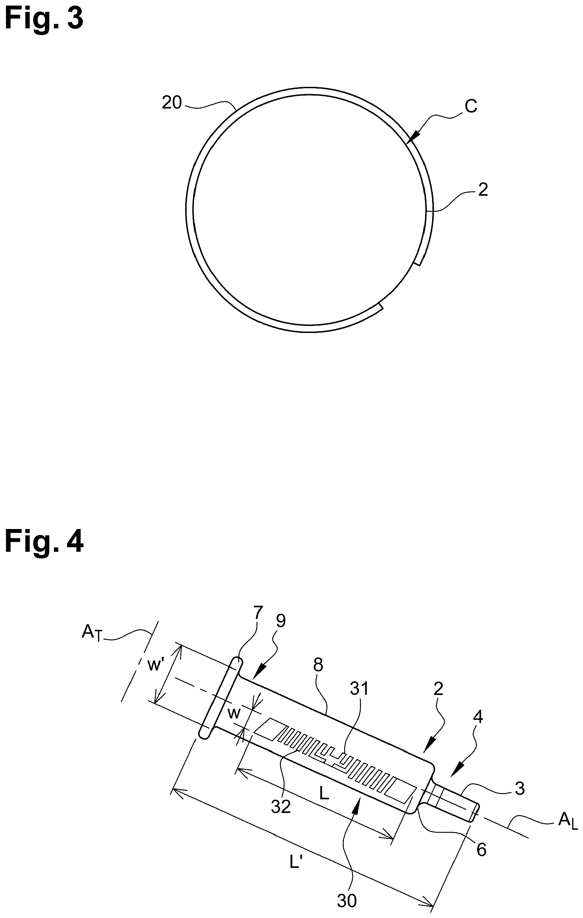

The RFID tag 20 may extend along a longitudinal axis A_{L} of said barrel 2. In this example, the RFID tag 20 has a length L extending over at least 15% of a length L' of the barrel 2. The RFID tag 20 may have a width W extending between 10 and 100% of a circumference W' of an external wall 8 of the barrel 2, 100% being excluded (W' being represented on Figure 3).

The RFID tag 20 can be for example located at a proximal end 9 of said barrel 2.

As shown in Figure 4, the barrel 2 comprises a RFID tag 30 located on the barrel 2, said RFID tag 30 comprising one RFID antenna 32 made of a substantially transparent and conductive ink. The RFID tag 30 may comprise a RFID chip 31 connected to the RFID antenna 32.

The RFID tag 30 extends along the longitudinal axis A_{L} of said barrel 2. In this example, the RFID tag 30 has a length L extending over at least 70% of a length L' of a length of the barrel 2. The RFID tag 30 may have a width W extending about 15% of the circumference W' of the side wall 8 of the barrel 2.

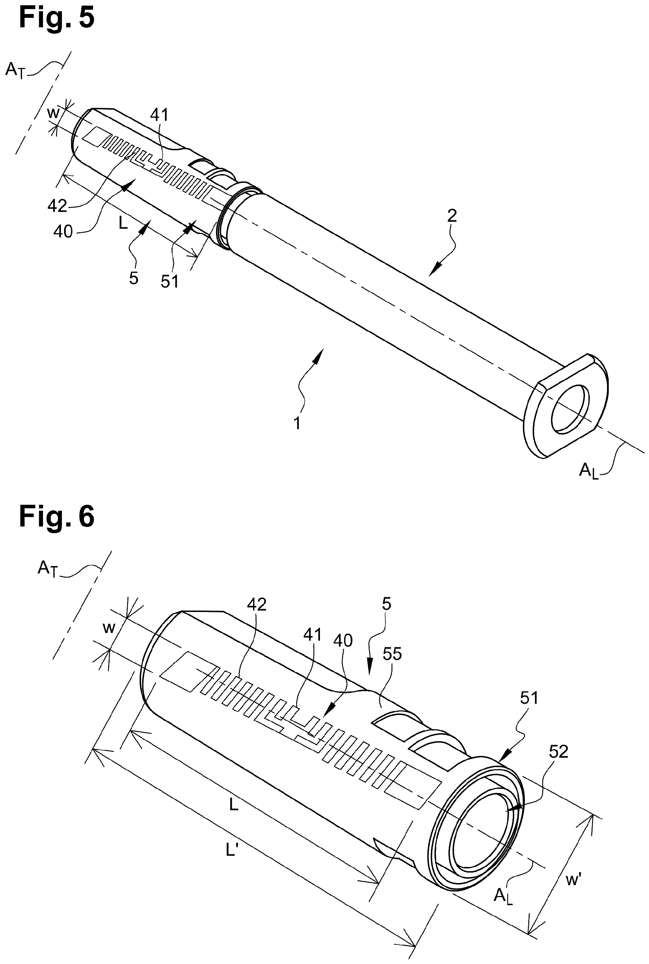

Figures 5 and 6 illustrate an embodiment wherein the component having at least one substantially transparent portion is the cover 5. The cover 5 comprises longitudinal axis A_{L} and a transversal axis A_{T}.

In an embodiment, the cover 5 comprises an outer casing 51 and an inner casing 52. The outer casing 51 is typically made of a rigid material while the inner casing 52 is made of a soft material. When the cover 5 is mounted on the hub portion 3 of the medical injection device 1, the needle is directly engaged with the inner casing 52. Preferably, the outer casing 51 is made of polypropylene (PP), polyethylene (PE), polyethylene terephthalate (PET), polystyrene (PS) or polycarbonate (PC), much preferably of polypropylene. Advantageously, the inner casing 52 is made of a deformable material such as TPE (Thermo Plastic Elastomer).

The cover 5 comprises a RFID tag 40, preferably located on an external surface of the cover 5, said RFID tag 40 comprising at least one RFID antenna 42 made of a substantially transparent and conductive ink. The RFID chip 41 may be connected to the RFID antenna 42. The RFID tag could also be located on an inner surface of the cover 5.

The RFID tag 40 may extend along the longitudinal axis A_{L} of said cover 5. In this example, the RFID tag 40 has a length L extending over at least 80% of a length L' of a length of the cover 5. The RFID tag 40 may have a width W extending about 15% of the circumference of a wall 55 of the cover 5.

Preferably, the method for manufacturing a medical component such as a syringe barrel 2 or cover 5 comprises a step A) including the application of the RFID antenna 22, 32, 42 on the medical component. In a step I), the medical component is provided. In a step II), the application of the RFID antenna 22, 32, 42 on the medical component is performed by printing, in particular by screen-printing or ink-jet printing. Preferably, a transparent and conductive ink is used to print the RFID antenna 22, 32, 42 on the medical component. Then, in a step II'), the bonding of the RFID chip 21, 31, 41 to said RFID antenna 22, 32, 42 may be performed by gluing.

In a step III), the RFID tag 20, 30, 40 may be directly topped by a protective transparent coating (not shown) to decrease the risk of removal or external damages. The protective transparent coating may be applied by serigraphy. For example, the protective transparent coating is made of an epoxy resin such as a varnish epoxy.

Alternatively, the method for the manufacture of the medical component comprises a step A) including the application of the RFID antenna 22, 32, 42 on the medical component. In a step i), the medical component is provided. In a step ii), the RFID antenna 22, 32, 42 may be applied on a transparent substrate (not shown) by printing, in particular the screen-printing or ink-jet printing. Preferably, a transparent and conductive ink is used to print the RFID antenna 22, 32, 42 on the transparent substrate. For example, the transparent substrate is made of plastic. Then, in a step ii'), the application of the RFID chip 21, 31, 41 to said RFID antenna 22, 32, 42 may be performed by gluing. In a step iii), the medical component is provided. In a step iv), the implementation of the RFID tags 20, 30, 40 into the medical component may be performed by over molding or in mold labelling.

In a step v), the RFID tag 20, 30, 40 may be directly topped by a protective transparent coating (not shown) to decrease the risk of removal or external damages. For example, the protective transparent coating is made of an epoxy resin such as a varnish epoxy.

In the illustrated examples, the RFID chip 21, 31, 41 may be a printed circuit. The RFID chip 21, 31, 41 may have a length equal to 0.2mm, a width equal to 0.2mm and a thickness equal to 0.05mm. Thus, the visual impact of the medical injection is limited.

The RFID tag 20, 30, 40 can be for example a LF-RFID tag, a HF-RFID tag or a UHF-RFID tag.

The medical component according to the present invention allows a highly effective individual identification of a medical injection device comprising said component, with a reduced impact on visual inspection, with few or no risks of being removed or damaged, and with a limited impact on the manufacturing process.

## Claims

1. A medical component comprising a Radio Frequency Identification RFID tag (20, 30, 40) located on the component, said RFID tag (20, 30, 40) comprising at least one RFID antenna (22, 32, 42) made of a substantially transparent and conductive ink.

2. Medical component according to claim 1, wherein the medical component is chosen from: a barrel (2) having a hub portion (3) provided at distal end, a plunger rod, a plunger stopper and a cover (5).

3. Medical component according to anyone of the preceding claims, wherein a RFID chip (21, 31, 41) is connected to the at least one RFID antenna (22, 32, 42) being made of a substantially transparent and conductive ink.

4. Medical component according to anyone of the preceding claims, wherein the RFID tag (20, 30, 40) has a length (L) extending over at least 15%, typically at least 50%, preferably at least 70% of a length (L') of said component.

5. Medical component according to anyone of the preceding claims, wherein the RFID tag (20, 30, 40) is directly covered by a protective transparent coating.

6. Medical component according to anyone of the preceding claims, wherein the RDIF tag (20, 30, 40) is applied on a transparent substrate.

7. Medical injection device (1) comprising a medical component according to anyone of the preceding claims.

8. Method for manufacturing a medical component according to anyone of claims 1 to 6, comprising a step A) wherein a RFID tag (20, 30, 40) comprising at least one RFID antenna (22, 32, 42) made of a transparent and conductive ink is applied onto a medical component.

9. Method according to claim 8, wherein the step A) comprises the following steps:
I. The provision of the medical component and
II. The application onto the medical component of the RFID tag (20, 30, 40) comprising at least one RFID antenna (22, 32, 42) made of a transparent and conductive ink.

10. Method according to claim 9, wherein in step II), the application of the at least one RFID antenna (22, 32, 42) made of a transparent and conductive ink onto the component is performed by printing, in particular by screen-printing or ink-jet printing; by roll coating; by blade coating; by rod coating, by spin coating or pad-printing or by slot-die coating.

11. Method according to anyone of claims 9 or 10, further comprising a step II') performed after step II), wherein a RFID chip (21, 31, 41) is bonded to said at least one RFID antenna (22, 32, 42) made of a transparent and conductive ink.

12. Method according to claim 11, wherein the bonding of the RFID chip (21, 31, 41) to said at least one RFID antenna (22, 32, 42) made of a transparent and conductive ink is performed by gluing, by brazing or by welding.

13. Method according to anyone of claims 9 to 12, further comprising a step III) wherein a protective transparent coating is applied onto the RFID tag (20, 30, 40) by serigraphy.

14. Method according to claim 13, wherein the step A) comprises the following steps:
i) the provision of a transparent substrate;
ii) the application onto the transparent substrate of the RFID tag (20, 30, 40) comprising at least one RFID antenna (22, 32, 42) made of a transparent and conductive ink;
iii) the provision of the medical component and
iv) the application onto the medical component of the transparent substrate comprising the RFID tag (20, 30, 40) comprising at least one RFID antenna (22, 32, 42) made of a transparent and conductive ink.

15. Method according to claim 14 wherein in step iv), the RFID tag (20, 30, 40) comprising at least one RFID antenna (22, 32, 42) made of a transparent and conductive ink is implemented onto the component by over molding or in mold labelling.
